# EUROPEAN PATENT APPLICATION

(11) **EP 4 685 472 A1**
(43) Date of publication of application: **28.01.2026**
(21) Application number: 24773877.6
(22) Date of filing: 29.02.2024
(51) Int. Cl.: G01N 27/22, A61F 13/42

(54) **CAPACITIVE DOUBLE-SIDED SENSING FILM, RELATED PRODUCT, APPARATUS AND METHOD**

(30) Priority: 22.03.2023 CN 202310279525
(71) Applicant: Ediaper Technology Limited, N.T., Hong Kong (HK); Shanghai Eway Ediaper technology Co., Ltd., Minhang District, Shanghai, China 200241 (CN)
(72) Inventor: WONG, Sun Hoi, Hong Kong (CN); XU, Fei, Hong Kong (CN); CHEN, Zhen, Hong Kong (CN)
(74) Representative: Gulde & Partner
(86) International application number: PCT/CN2024/079198
(87) International publication number: WO 2024/193309

(57) **Abstract**

The present invention provides a capacitive double-sided sensing film, comprising a plastic thin film substrate, and a first detection electrode and a second detection electrode which are respectively arranged on the upper surface and the lower surface of the plastic thin film substrate; the first detection electrode and the second detection electrode respectively comprise a first metal evaporative deposition layer and a second metal evaporative deposition layer; the first and second metal evaporative deposition layers have the same size as that of the plastic thin film substrate, and overlap each other to collectively form a parallel plate capacitor and to generate a first capacitance for providing information related to the area of the sensing film. When at least part of the upper and lower surfaces of the sensing film are impregnated with excrement and are thus connected together, a second capacitance positively correlated with the impregnation area is generated between the first detection electrode and the second detection electrode, providing state information about the impregnation of the excrement. Further provided in the present invention are a smart diaper comprising a capacitive double-sided sensing film, a moisture state monitoring apparatus comprising a smart diaper, a manufacturing method for a capacitive double-sided sensing film, and a method for measuring the saturation of and distinguishing between excrement and urine in a smart diaper.

## Description

This application claims priority to Chinese Patent Application No. 202310279525.2, filed on March 22, 2023, entitled "A Double Electric Layer Capacitive Film Sensor and Related Products, Devices, and Methods", the entire contents of which are incorporated herein by reference.

### TECHNICAL FIELD

The present invention relates to a sensing film, and more particularly, to a capacitive double-sided sensing film for implementing excrement detection, its manufacturing method, and related applications.

### BACKGROUND

The sensing film referred to in this application is a film with moisture detection functionality that can be used as a raw material for producing smart diapers. By embedding it within a diaper during production, the diaper becomes a smart diaper capable of providing quantified wetness status information and excrement information. This has practical significance for the scientific use and changing of diapers and is an important direction for diaper development.

Regarding prior art, Chinese Patent Publication No. CN 114324502 B discloses a capacitive sensing film and related smart diaper and detection system device. Its sensing film includes a metal film electrode (detection electrode), a flexible substrate, and a waterproof protective layer, wherein the detection electrode is disposed on the flexible substrate, and the waterproof protective layer covers the detection electrode for protection. When the sensing film is wetted by a liquid containing electrolytes, it can achieve wetness detection functionality based on electrolytic capacitance.

Although the aforementioned prior art can achieve quantified wetness detection functionality, its two electrodes are arranged side-by-side on the same surface of the flexible substrate, and include a gap (blank area) between them. This structure increases the width of the sensing film. When the sensing film is disposed between the top sheet and the absorbent core of a diaper, it can affect the penetration of urine from the top sheet to the absorbent core. Furthermore, since the two electrodes in the prior art lack overlapping portions, the initial capacitance between the electrodes cannot be used to determine the specification parameters of the sensing film, thus preventing adaptive operation or damage compensation. It also cannot solve the problem of capacitive interference caused by one-sided wetness or one-sided human body contact.

Additionally, the sensing film of the prior art cannot be arbitrarily cut for use as needed, as this would destroy the structural relationship between the two electrodes. Moreover, the blank area between electrodes typically requires patterning processes (e.g., laser etching, chemical etching, aluminum washing) to create, which significantly increases production costs. For example, vacuum-metallized film with patterns often costs several times more than fully metallized film without patterns.

Furthermore, the electrodes in the prior art are protected on both sides by the flexible substrate and the waterproof protective layer. The connection terminals of the detection device cannot make face-to-face contact and electrical connection with the electrodes. If electrical connection is to be made through exposed portions without the waterproof protective layer, production would require tracking and positioning these exposed portions and placing them at specific locations on the diaper, undoubtedly increasing production costs, usage costs, and reducing the versatility of the sensing film.

The aforementioned deficiencies in the prior art necessitate new technical solutions.

### SUMMARY OF THE INVENTION

The technical problem to be solved by the present invention is to provide a capacitive double-sided sensing film with low internal resistance, good flexibility, compatibility with conventional vacuum metallization processes, reduced membrane width, elimination of single-sided interference, ease of cutting and use, capability for electrical connection at any position, ability to provide its own specification parameter information and wetness/impregnation status information, along with its manufacturing method, a smart diaper containing the membrane, a monitoring system device, and a method for implementing saturation detection and urine/feces differentiation in smart diapers.

To address the above technical problem, an embodiment of the present invention provides a capacitive double-sided sensing film for excrement detection. The membrane comprises a plastic film substrate, and a first detection electrode and a second detection electrode respectively disposed on upper and lower surfaces of the plastic film substrate. The first and second detection electrodes respectively comprise a first metal electrode and a second metal electrode, configured to reduce electrode internal resistance and capacitance value between the electrodes and liquid, thereby reducing the time constant of the detection circuit. The first and second metal electrodes respectively comprise a first metal metallization layer and a second metal metallization layer, configured to reduce electrode thickness via a metal metallization process to provide required flexibility. The metal metallization layers are complete and integral vacuum metallization layers that completely cover the upper and lower surfaces of the plastic film substrate, configured to be compatible with conventional vacuum metallization processes and save costs by eliminating the need to generate circuits with specific patterns. The first and second metal metallization layers are located on the upper and lower surfaces of the plastic film substrate, respectively, and are arranged overlapping each other, configured to reduce the width of the capacitive double-sided sensing film and prevent capacitive interference caused by single-sided wetness or human body single-sided contact. The first metal metallization layer, the second metal metallization layer, and the plastic film substrate are identical in size and mutually overlapping, configured to facilitate cutting for use, such that any cutting does not alter the identical size and mutually overlapping structural relationship. The first detection electrode, the second detection electrode, and the plastic film substrate together further constitute a parallel plate capacitor and generate a first capacitance, configured to provide information related to the area of the capacitive double-sided sensing film, determine the range and scale of signal output, thereby facilitating adaptive operation and implementing damage compensation. When excrement containing electrolytes wets and connects at least a portion of the upper and lower surfaces of the sensing film together, a second capacitance positively correlated with the wetted area is generated between the first and second detection electrodes, configured to provide status information about the sensing film being wetted by the excrement.

In one embodiment, the magnitude of the first capacitance is proportional to the area and dielectric constant of the plastic film substrate and inversely proportional to the thickness of the plastic film substrate, and is independent of the shape of the capacitive double-sided sensing film and its wetting state.

The second capacitance is a series value of a capacitance generated between the first detection electrode and the excrement and a capacitance generated between the second detection electrode and the excrement, configured to ascertain the excrement wetting state of the capacitive double-sided sensing film and the nature of the excrement based on analysis of the magnitude and variation pattern of the series value.

The metal metallization layers contain no covering layers or holes that affect their conductivity, allowing electrical connection with a capacitance detection device at any position, thereby improving the versatility and ease of use of the capacitive double-sided sensing film.

In another embodiment, the membrane comprises a semiconductor covering layer or an insulating covering layer covering an outer surface of any one of the metal metallization layers, configured to reduce the capacitance value between the respective metal metallization layer and the excrement, thereby achieving directional excrement detection functionality focused towards the covering layer direction. Alternatively, the membrane comprises a conductive covering layer having a multi-microporous structure or a rough surface covering an outer surface of any one of the metal metallization layers, configured to increase the capacitance value between the respective metal metallization layer and the excrement, thereby achieving directional excrement detection functionality focused towards the non-covering layer direction. Alternatively, the membrane comprises a locally thickened conductive layer covering a specific position on the outer surface of any one of the metal metallization layers, configured to increase the thickness of conductive material at that location, so that when electrical connection is made at that location with the metal metallization layer by means of a metal tip piercing method, additional conductivity can be provided in the thickness direction to improve the reliability of the electrical connection.

In a further embodiment, the semiconductor covering layer comprises a metal oxide metallization layer, the insulating covering layer comprises an insulating material coating or metallization layer, both configured to prevent charged ions in the excrement from directly contacting the outer surface of the metal metallization layer, thereby reducing the generation of the second capacitance. The conductive covering layer comprises a carbon-based conductive ink covering layer or a graphene metallization layer, configured to increase the surface area in contact with the excrement, thereby increasing the generation of the second capacitance. The locally thickened conductive layer comprises a conductive ink coating or printed layer.

In yet another embodiment, the metal metallization layer comprises a vacuum aluminum metallization layer. The plastic film substrate comprises a polyester film, polyethylene film, polypropylene film, or polyimide film. The excrement comprises urine, feces, sweat, or blood.

Another embodiment of the present invention provides a smart diaper. The smart diaper comprises the capacitive double-sided sensing film according to the first embodiment and a disposable absorbent article. The disposable absorbent article comprises a top sheet, an absorbent core, and a backsheet, sequentially stacked and bonded. The capacitive double-sided sensing film is disposed above the top sheet or between the top sheet and the backsheet, and is configured to provide information related to the wetting state of the disposable absorbent article through the second capacitance.

In one embodiment of the smart diaper, the area or length of the capacitive double-sided sensing film has a corresponding relationship with the model specification or length of the disposable absorbent article, configured to ascertain the model specification information or length information of the disposable absorbent article by detecting the first capacitance, thereby facilitating adaptive operation.

In another embodiment of the smart diaper, the capacitive double-sided sensing film is disposed between the top sheet and the backsheet, and an adhesive-free area is included between the capacitive double-sided sensing film and the top sheet or the backsheet, to facilitate electrical connection of one or both sides of the capacitive double-sided sensing film with connection terminals of a capacitance detection device, and to enable excrement detection functionality of the disposable absorbent article through the capacitance detection device. Alternatively, an opening is included on the top sheet or the backsheet, allowing one or both sides of the capacitive double-sided sensing film to be exposed through the opening and electrically connected to the connection terminals of the capacitance detection device, thereby enabling excrement detection functionality of the disposable absorbent article through the capacitance detection device.

In a further embodiment of the smart diaper, the capacitive double-sided sensing film is disposed between the top sheet and the absorbent core. The absorbent core comprises a polymer absorbent material configured to gradually absorb and lock moisture entering from the top sheet, causing the top sheet to gradually become dry, such that the top sheet experiences a change process from wet to dry during each urination process, while simultaneously causing the second capacitance to experience a change process from large to small. The speed at which the second capacitance changes from large to small is related to the saturation of the absorbent core; the higher the saturation, the slower the speed at which the second capacitance changes from large to small. The saturation of the absorbent core can be ascertained based on the speed at which the second capacitance changes from large to small. When the excrement is loose stool, since loose stool is difficult for the absorbent core to absorb, it will cause the second capacitance value to remain high for a long time. This characteristic can be used to determine that the excrement is loose stool.

Another embodiment of the present invention provides a smart diaper status monitoring device. The device comprises the smart diaper according to the aforementioned smart diaper embodiments and a capacitance detection device. The capacitance detection device comprises at least two connection terminals electrically connected to the first detection electrode and the second detection electrode of the capacitive double-sided sensing film, respectively, and is configured to implement excrement status monitoring functionality.

In one embodiment of the monitoring device, it comprises a wireless transmitter and a wireless receiver, configured to transmit and receive excrement status information of the smart diaper, thereby implementing wireless status monitoring and status display functionality for the smart diaper.

Another embodiment of the present invention provides a method for manufacturing the capacitive double-sided sensing film according to the first embodiment. The method comprises the steps of: using any one of polyester film, polyethylene film, polypropylene film, or polyimide film as the plastic film substrate, wherein the plastic film substrate is in the form of a wide-width web; using any one of aluminum, copper, gold, silver, zinc, or chromium as the metal material to be metallized; metallizing the metal material onto one surface of the plastic film substrate as the first metal metallization layer by vacuum metallization technology to generate a single-sided metal metallized film, and then winding it into a wide-width single-sided metal metallized film web; metallizing the metal material onto the non-metallized surface of the single-sided metal metallized film web as the second metal metallization layer by vacuum metallization technology to generate a double-sided metal metallized film, and then winding it into a wide-width double-sided metal metallized film web; slitting and rewinding the wide-width double-sided metal metallized film web according to a required width to generate a plurality of narrow-width double-sided metal metallized film webs, wherein the upper and lower surfaces of the plastic film substrate are completely covered by the metal material, and the first metal metallization layer, the second metal metallization layer, and the plastic film substrate are identical in size and shape and are arranged overlapping in the thickness direction.

In one embodiment of the method, prior to slitting and rewinding the wide-width double-sided metal metallized film web, the method further comprises a step of providing an insulating covering layer, a semiconductor covering layer, or a conductive covering layer on one surface of the wide-width double-sided metal metallized film web by vacuum metallization, printing, or coating process.

In a further embodiment of the method, the insulating covering layer comprises a polymer material coating or a silicon dioxide metallization layer; the semiconductor covering layer comprises an aluminum oxide vacuum metallization layer; the conductive covering layer comprises a carbon-based conductive ink coating, printed layer, or a graphene metallization layer.

Another embodiment of the present invention provides a method for implementing saturation detection and urine/feces differentiation in the smart diaper according to the embodiment where the sensing film is between the top sheet and absorbent core and describes capacitance decay. The method comprises the steps of: electrically connecting a first signal connection terminal and a second signal connection terminal of a capacitance detection device to the first detection electrode and the second detection electrode of the capacitive double-sided sensing film of the smart diaper, respectively; setting five thresholds, namely a first threshold, a second threshold, a third threshold, a fourth threshold, and a fifth threshold; obtaining, by the capacitance detection device, the second capacitance related to the excrement status of the smart diaper from the capacitive double-sided sensing film; comparing the variation pattern of the second capacitance with the thresholds, and judging the wetness status of the smart diaper and the nature of the excrement based on the comparison result; judging that an excretion process is occurring when the rate of increase of the second capacitance is greater than the first threshold; judging that the excretion process has ended when the second capacitance falls back from a peak value; judging that the excrement is urine and the smart diaper is not yet saturated when the fallback speed of the second capacitance is greater than the second threshold; judging that the smart diaper isapproaching saturation when the fallback speed of the second capacitance is less than the third threshold; judging that the excrement is loose stool when the fallback speed of the second capacitance is less than the fourth threshold or when it does not fall back within a preset time period; and judging that the excrement is a mixture of urine and stool when the fallback speed of the second capacitance is first fast and then slow, and the speed difference between the two is greater than the fifth threshold.

The beneficial effects of the present invention include: 1. The detection electrodes are metal electrodes with smooth, flat surfaces and good conductivity, effectively reducing electrode internal resistance and the capacitance value between the electrodes and liquid, thereby reducing the time constant of the detection circuit and increasing detection speed. 2. The metal electrodes are formed by vacuum metallization, effectively reducing the thickness of the metal layer to provide the required flexibility. 3. The metal metallization layers are complete and integral vacuum metallization layers, compatible with conventional vacuum metallization processes, and save costs by eliminating the need for patterning processes to generate circuits with specific shapes. 4. The two metal metallization layers are located on the upper and lower surfaces of the plastic film substrate in an overlapping arrangement, effectively reducing the width of the sensing film and preventing capacitive interference caused by single-sided wetness or human body single-sided contact. 5. The metal metallization layers and the plastic film substrate are identical in size and mutually overlapping, allowing arbitrary cutting for use. 6. The detection electrodes and the plastic film substrate together constitute a parallel plate capacitor and generate a first capacitance, providing information related to the area of the sensing film, determining the range and scale of signal output, enabling damage compensation, and facilitating adaptive operation. 7. The metal metallization layers have no covering layers or holes affecting conductivity and connectivity, allowing electrical connection with a detection device at any position, improving the versatility and ease of use of the sensing film.

The capacitive double-sided sensing film of the present invention is particularly suitable for smart diaper production, providing a simple, convenient, effective, and low-cost solution for excrement detection in diapers, creating favorable conditions for the intelligent upgrade of traditional diapers.

### BRIEF DESCRIPTION OF THE DRAWINGS

To describe the technical solutions in the embodiments of the present invention more clearly, the following briefly introduces the accompanying drawings required for describing the embodiments. Obviously, the accompanying drawings in the following description show merely some embodiments of the present invention, and a person of ordinary skill in the art may still derive other drawings from these accompanying drawings without creative efforts.

FIG. 1 is a perspective structural schematic diagram of a capacitive double-sided sensing film according to an embodiment of the present invention.
FIG. 2 is a schematic A-A' cross-sectional view and an equivalent circuit diagram of the sensing film in an initial/dry state according to an embodiment of the present invention.
FIG. 3 is a schematic A-A' cross-sectional view and an equivalent circuit diagram of the sensing film immersed in a liquid containing electrolytes according to an embodiment of the present invention.
FIG. 4 is a simplified schematic A-A' cross-sectional view and an equivalent circuit diagram of the sensing film immersed in a liquid containing electrolytes according to an embodiment of the present invention.
FIG. 5 is a plan view structural schematic diagram of the sensing film including damages such as pinholes, cracks, and notches according to an embodiment of the present invention.
FIG. 6 is a system structural schematic diagram of a smart diaper according to an embodiment of the present invention, formed by placing the sensing film within a disposable absorbent article/diaper.
FIG. 7 is an exploded perspective structural schematic diagram of the smart diaper according to an embodiment of the present invention, formed by placing the sensing film between the top sheet and the absorbent core of the diaper.
FIG. 8 is a structural schematic diagram of the smart diaper according to an embodiment of the present invention, including a gap between the backsheet and the sensing film for capacitance signal output.
FIGS. 9A and 9B are system structural schematic diagrams of the smart diaper electrically connected to a capacitance detection device according to an embodiment of the present invention.
FIG. 10 is a schematic diagram of the smart diaper containing excrement according to an embodiment of the present invention.
FIG. 11 is a schematic B-B' longitudinal sectional view and an equivalent circuit diagram of the smart diaper containing high-fluidity excrement (e.g., urine) according to an embodiment of the present invention.
FIG. 12 is a schematic B-B' longitudinal sectional view of the smart diaper containing low-fluidity excrement (e.g., loose stool) according to an embodiment of the present invention.
FIG. 13 is a schematic C-C' cross-sectional view of the smart diaper containing low-fluidity excrement (e.g., loose stool) according to an embodiment of the present invention.
FIG. 14 is a cross-sectional structural schematic diagram of the sensing film including covering layers that alter detection sensitivity on the first and second metal metallization layers according to an embodiment of the present invention.
FIG. 15 is a system block diagram of a smart diaper status monitoring device formed by the smart diaper, a capacitance detection device, and a wireless receiving device according to an embodiment of the present invention.
FIG. 16 is a flowchart of a method for manufacturing the sensing film according to an embodiment of the present invention.
FIG. 17 is a flowchart of a method for implementing saturation detection and urine/feces differentiation on the smart diaper according to an embodiment of the present invention.

### DETAILED DESCRIPTION

The following descriptions of the various embodiments are provided with reference to the accompanying drawings to illustrate specific embodiments in which the present invention may be practiced. The directional or positional terms mentioned in the present invention, such as "upper", "lower", "front", "rear", "left", "right", "inner", "outer", "top", "bottom", "side", etc., are only directions or positions with reference to the accompanying drawings. Therefore, the used directional or positional terms are for explaining and understanding the present invention, rather than limiting the scope of protection of the present invention.

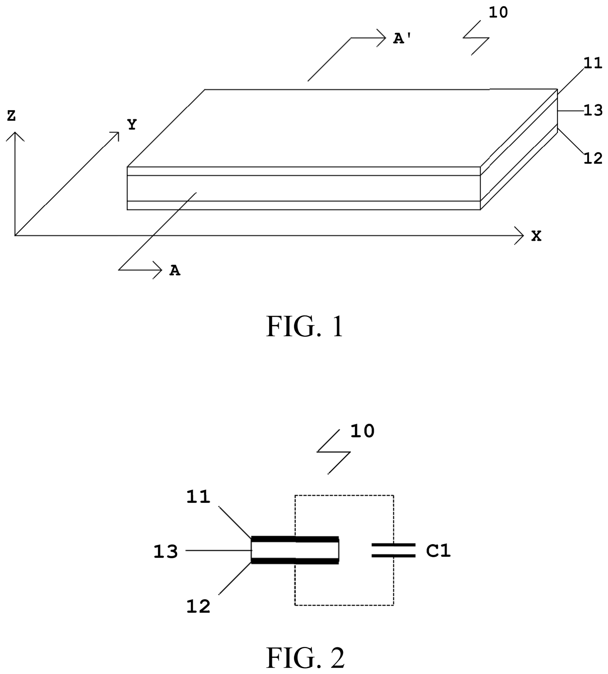

The present invention is further described below with reference to the accompanying drawings. Referring to FIG. 1, this is a perspective structural schematic diagram of a capacitive double-sided sensing film (abbreviated as capacitive sensing film, double-sided sensing film, sensing film) 10 according to an embodiment of the present invention. The membrane 10 appears rectangular in the figure but can have various shapes in practical applications. For ease of viewing, the various components of the membrane 10 are drawn relatively thick; in practice, these components are very thin. The sensing film is a single-substrate film that is not only thin but also very soft and can be cut for use according to practical application needs.

The sensing film 10 comprises a flexible, waterproof, insulating, and very thin plastic film substrate 13, and a first detection electrode 11 and a second detection electrode 12 respectively disposed on the upper and lower surfaces of the plastic film substrate 13. Theoretically, any conductive substance, including metal, carbon black, graphene, conductive ink, etc., can be used as the detection electrode. In this invention, electrodes made of metal are called metal electrodes, electrodes made of carbonaceous substances are called carbon electrodes, and electrodes printed with conductive ink are called printed electrodes.

Metal electrodes can be generated not only by metal foil or metal electroplating but also by vacuum metal metallization, i.e., the metal electrodes comprise metal metallization layers. In this case, the first detection electrode 11 and the second detection electrode 12 can also be called the first metal electrode 11 and the second metal electrode 12, or the first metal metallization layer 11 and the second metal metallization layer 12. Metal electrodes made from metal foil or electroplating are usually thicker and stiffer, making them less suitable for use in absorbent hygiene products. Therefore, this invention preferably uses metal metallization layers to form the detection electrodes. In the following embodiments, the first and second detection electrodes are mainly described as the first and second metal metallization layers.

The metal metallization layers can be generated by physical vapor deposition methods, such as vacuum metallization or RF sputtering, which deposit a layer of metal on the upper and lower surfaces of the plastic film substrate. This layer not only has good conductivity but is also very thin, typically between 20 and 200 nanometers, and possesses good flexibility.

Common metal metallization materials include aluminum, copper, gold, silver, zinc, chromium, etc., with aluminum being the most common. Common flexible plastic film substrates include polyester film (PET), polypropylene film (CPP, BOPP), polyethylene film (PE), polyimide film (PI), etc., with thicknesses generally between 1 and 100 micrometers. A PET film substrate 3-12 micrometers thick with an aluminum metallization layer 50-100 nanometers thick is preferred, as the resulting double-sided aluminized film offers excellent cost-performance.

In FIG. 1, the X-axis is the length direction (longitudinal direction) of the sensing film, the Y-axis is the width direction (transverse direction), and the Z-axis is the thickness direction. Conventionally, the two surfaces in the thickness direction are called the upper and lower surfaces; the two edges in the length direction are called the ends, and the cross-section in the length direction is called the longitudinal section; the two edges in the width direction are called the sides, and the cross-section in the width direction is called the transverse section.

The first metal metallization layer 11, the second metal metallization layer 12, and the plastic film substrate 13 of this embodiment are completely identical in size and shape and are arranged overlapping in the thickness direction Z (i.e., their orthographic projections overlap). The metal metallization layers 11, 12 disposed on the upper and lower surfaces of the sensing film completely cover the plastic film substrate 13. They are smooth, flat, integral metal metallization layers without any specific patterns or blank areas. This sensing film is not only low-cost but also highly versatile. Since there are no covering layers, holes, or blank areas affecting conductivity on the metal metallization layers, electrical connection can be made with the connection terminals of a detection device anywhere, eliminating the need for any positioning treatment during use.

This type of sensing film, completely covered by metal metallization layers, is the easiest to produce. It can be generated simply by feeding the plastic film substrate into a conventional vacuum metallizer for metallization, meaning it is compatible with traditional vacuum metallization processes. It does not require any physical or chemical methods (e.g., localized metallization, laser etching, chemical etching/aluminum washing) to create the required circuit patterns or blank areas, effectively reducing the production cost of the sensing film.

The first metal metallization layer 11, the second metal metallization layer 12, and the plastic film substrate 13 together form a parallel plate capacitor. The first and second metal metallization layers 11, 12 serve as the two electrodes of the parallel plate capacitor, and the plastic film substrate 13 serves as the dielectric. The capacitance generated is called the parallel plate capacitance, initial capacitance, or first capacitance (C1). The magnitude of C1 is proportional to the area of the plastic film substrate, proportional to the dielectric constant of the plastic film substrate, and inversely proportional to the thickness of the plastic film substrate. It is independent of the shape of the sensing film and its wetting state. The A-A' cross-sectional view and equivalent circuit diagram in the dry state are shown in FIG. 2.

The initial capacitance/first capacitance C1 is inherent to the sensing film. Once the area of the sensing film, the material of the plastic film substrate, and its thickness are determined, C1 is determined and remains constant, unrelated to the wetting state of the membrane. Due to this characteristic, in practical applications, detecting C1 can allow reverse calculation of the sensing film's area, thus ascertaining its specification parameters. For example, knowing the width allows determining the length information of the sensing film, and through the length information, the specification information of the diaper can be ascertained, enabling adaptive operations for different diaper specifications, including automatic setting of detection range and scale, automatic setting of triggering thresholds for diaper change alerts/alarms, etc.

The liquid/excrement detection capability of the sensing film is achieved through capacitance generated when the membrane is wetted by a liquid/excrement containing electrolytes (abbreviated as liquid). Among these capacitances, the electric double-layer capacitance is most important and is the main component of the second capacitance. According to electric double-layer capacitance theory, when a liquid containing electrolytes contacts a conductive solid (detection electrode), an electric double-layer capacitance is generated at the interface between the solid and the liquid. When a DC voltage is applied between the electrodes, anions in the liquid accumulate at the positive electrode, and cations accumulate at the negative electrode. The ions in the liquid and the opposite ions on the solid electrode form a layer of ionic dielectric, thus generating the electric double-layer capacitance.

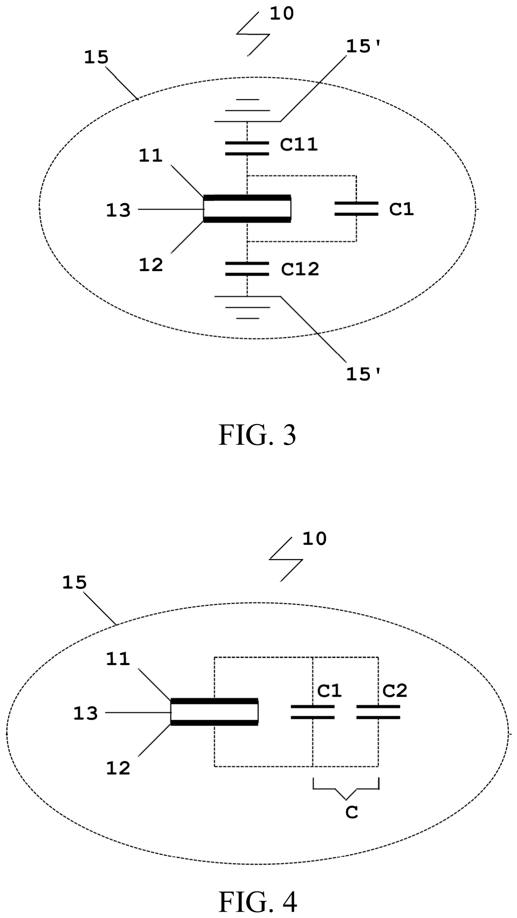

As shown in FIG. 3, when the sensing film 10 is immersed in a liquid 15 containing electrolytes (including tap water, physiological saline, urine/loose stool/sweat/menstrual blood containing salts, etc.), in addition to the initial capacitance/first capacitance C1 generated by the membrane's own structure between the first and second metal metallization layers 11, 12, an electric double-layer capacitance C11 is generated between the first metal metallization layer 11 and the liquid 15, and an electric double-layer capacitance C12 is generated between the second metal metallization layer 12 and the liquid 15.

Since the liquid containing electrolytes is conductive, the potential 15' within the conductive liquid is equal everywhere and is considered ground potential in the figure. It automatically connects capacitance C11 and capacitance C12 in series. Therefore, the capacitance C2 generated between the first metal metallization layer 11 and the second metal metallization layer 12 due to the liquid is the series value of C11 and C12, i.e., C2 = C11 x C12 / (C11 + C12). This series value C2 is referred to as the second capacitance.

It is important to note that the second capacitance C2 is only generated if both C11 and C12 are non-zero. If the value of either C11 or C12 is zero, the series value of the two capacitances will also be zero. This means C2 is only generated when at least a portion of both the first and second metal metallization layers 11, 12 are simultaneously wetted by and connected through the liquid 15.

The equivalent circuit diagram obtained by simplifying the circuit in FIG. 3 is shown in FIG. 4. In FIG. 4, the total capacitance C (abbreviated as capacitance C) generated between the first and second metal metallization layers 11, 12 is the sum (parallel value) of the first capacitance C1 and the second capacitance C2. After detecting capacitance C, the value of the second capacitance C2 can be obtained by C2 = C - C1. Since C2 is usually much larger than C1 in the wetted state, C1 can often be neglected, and C2 can be considered approximately equal to C in such cases.

The magnitude of the second capacitance C2 is positively correlated with the area of the first and second metal metallization layers 11, 12 that are wetted and connected by the liquid, thereby providing a quantitative liquid detection/wetness detection function. When the sensing film 10 is placed within a disposable absorbent article, it enables wetness detection functionality for the article.

The second capacitance C2 primarily consists of electric double-layer capacitance, also known as supercapacitance, which typically has a very large capacitance value. The larger the capacitance, the greater the time constant τ (time constant formula τ = R*C) of the detection circuit, and the longer the time required for one capacitance detection. In practical applications, to speed up detection, it is usually necessary to minimize the capacitance value of the electric double-layer capacitance between the two electrodes.

Different conductive materials have different abilities to generate electric double-layer capacitance in liquid. One reason this invention prefers metal metallization layers for the detection electrodes is that they have smooth surfaces, dense structures, and good conductivity, which not only reduces the generation of electric double-layer capacitance but also lowers the internal resistance of the electrodes, effectively reducing the time constant of the detection circuit and speeding up capacitance detection.

As for detection electrodes printed with carbon-based conductive ink, their surfaces are usually rough, and the carbon material 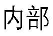 contains a large number of micropores, which create a large specific surface area leading to very large electric double-layer capacitance. Furthermore, the resistance of carbon electrodes is much higher than that of metal electrodes. These factors cause the time constant of the detection circuit formed by carbon electrodes to be much larger than that of metal electrodes.

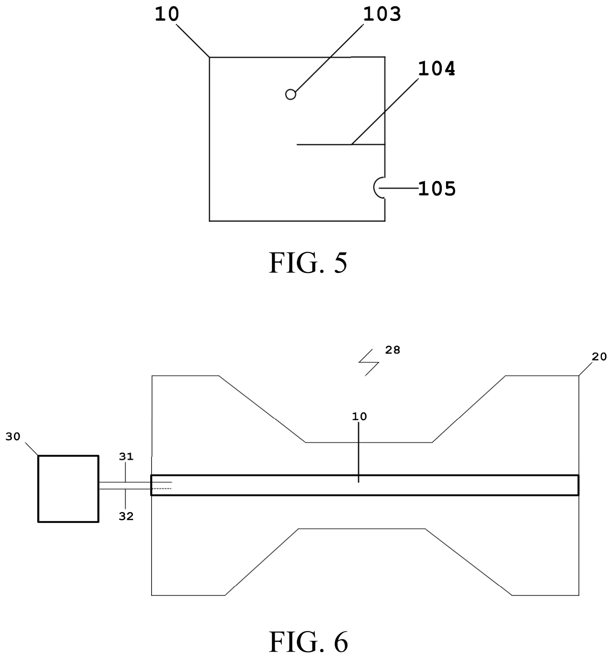

The sensing film can have various shapes and can be cut arbitrarily without affecting its performance, giving it excellent damage resistance. For example, in FIG. 6, the sensing film 10 has some damages, including pinholes 103, cracks 104, and notches 105. As long as these damages do not sever the membrane or significantly reduce the total effective area, the membrane can still function normally. Moreover, the total effective area of the membrane is measurable and proportional to the initial capacitance/first capacitance C1. Therefore, any damage can be assessed before use by detecting C1, and adjustments/compensation (damage compensation) can be made during use, reducing detection errors caused by damage.

The first capacitance C1 can also be used for adaptive operation. For a wetness detection system to know the degree of wetness (e.g., wetness percentage), it must know the signal output range and scale of the sensor in advance. For diaper wetness detection, since diapers come in various models/sizes, different sizes usually mean different absorption capacities, different sensing film areas, and different signal output ranges and scales. If the size specification parameters can be known in advance by detecting the first capacitance, adaptive operation can be implemented for diapers with different specifications.

The sensing film has excellent flexibility, making it particularly suitable for use in hygiene products/wearable products, such as for wetness detection in disposable absorbent articles/diapers. To facilitate diaper production, the sensing film can also be made in web form, with each roll being several kilometers long (preferably not less than 3000 meters) and widths ranging from a few millimeters to a few centimeters (preferably 0.5 to 1.5 cm), depending on needs.

Such webs suitable for diaper production can be generated by slitting from a large roll of wide-width sensing film several kilometers or even tens of kilometers long and several meters wide, and then rewinding onto circular cores or carrier tape reels to become narrow-width webs. During diaper production, simply placing the sensing film within the diaper allows the production of smart diaper products with quantitative excrement detection functionality.

FIG. 6 is a schematic diagram of an application of the sensing film in a disposable absorbent article. Disposable absorbent articles include diapers, diaper pads, training pants, underpads, sanitary napkins, and other absorbent hygiene products, with diapers being the most representative. The following description mainly uses diapers as an example, but the related descriptions also apply to other disposable absorbent articles. A diaper typically includes a top sheet (inner layer, dry layer, comprising hydrophilic nonwoven fabric), an absorbent core (absorption layer, comprising superabsorbent polymer SAP), a backsheet (leakproof layer, outer layer, comprising polyethylene film/PE film), and other main components, stacked and bonded together from top to bottom.

The wetness status of the diaper can be detected by a capacitance detection device (abbreviated as detection device, sensor) 30, which is usually a reusable external hardware device (can be called smart hardware or wearable device). It can be attached to the nonwoven fabric or front waist tape of the diaper to implement excrement detection functionality. The detection device 30 includes a first connection terminal 31 and a second connection terminal 32, which can be electrically connected to the first and second metal metallization layers of the sensing film, respectively, enabling capacitance detection between the first and second metal metallization layers. The wetness status of the diaper can be determined based on the capacitance value. In this embodiment, the sensing film 10 and the diaper 20 together constitute a smart diaper 28 (or smart absorbent article). Adding the detection device 30 forms a system device capable of monitoring the status of the smart diaper.

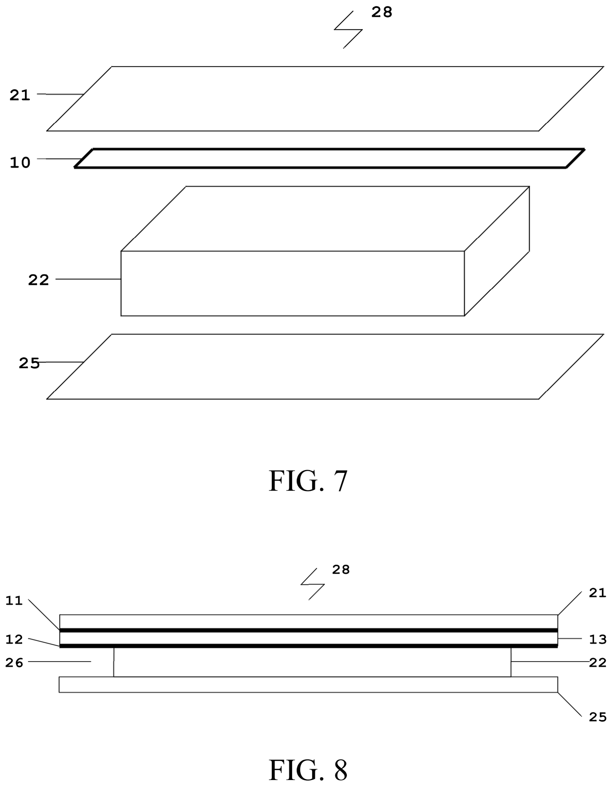

FIG. 7 is an exploded perspective structural schematic diagram of the smart diaper 28. To clearly show the relationship between the components, they are drawn separated; in practice, these components are bonded together. Reference numeral 21 is the top sheet of the diaper, 22 is the absorbent core, and 25 is the backsheet. In this embodiment, the sensing film 10 is disposed between the top sheet 21 and the absorbent core 22, focusing on monitoring the wetness status of the diaper's top sheet. During use, the top sheet is in direct contact with the user's skin, and its wetness status is consistent with the user's skin sensation.

In practical applications, the sensing film 10 can be placed anywhere/on any layer within the diaper. For example, besides between the top sheet 21 and the absorbent core 22, it can also be placed above the top sheet 21 of the diaper 20, or between the absorbent core 22 and the backsheet 25. The length of the sensing film 10 is usually consistent with the length of the diaper but can be longer or shorter as needed.

FIG. 8 is a schematic longitudinal sectional view of the smart diaper including a gap between the backsheet and the sensing film for capacitance signal output. It includes the top sheet 21, absorbent core 22, and backsheet 25 of the disposable absorbent article. The sensing film is disposed between the top sheet 21 and the absorbent core 22, with the first metal metallization layer 11 facing the top sheet 21 and the second metal metallization layer 12 facing the absorbent core 22.

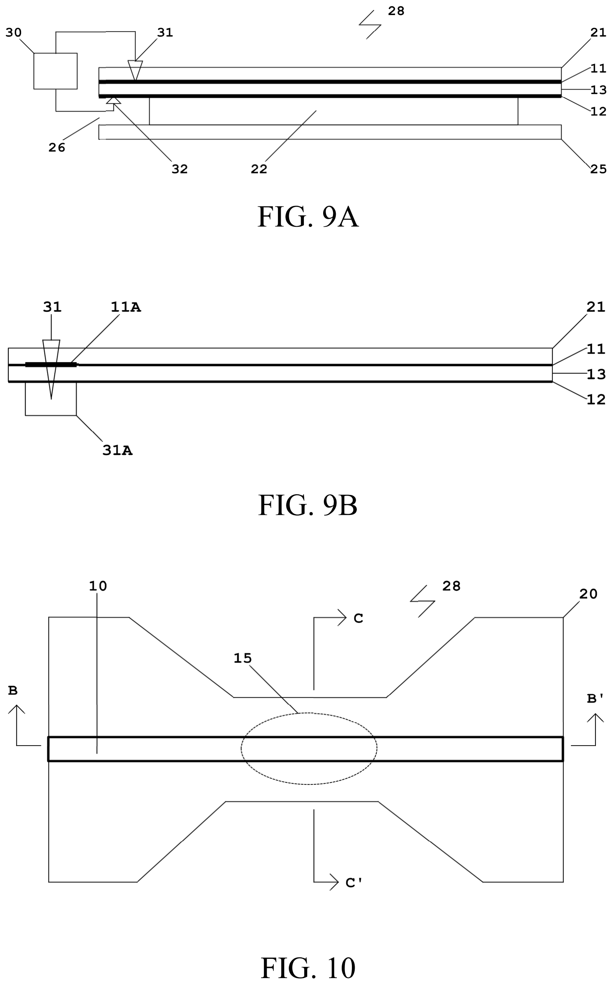

To output the signal from the second metal metallization layer 12, this embodiment includes a gap 26 between the second metal metallization layer 12 and the backsheet 25. The second connection terminal 32 of the detection device 30 can be inserted into this gap to make face-to-face contact and electrical connection with the second metal metallization layer 12. The first connection terminal 31 of the detection device 30 pierces the nonwoven top sheet 21 and makes electrical connection with the first metal metallization layer 11 underneath the nonwoven fabric. This allows the second capacitance generated between the first and second metal metallization layers 11, 12 to be output and transmitted to the detection device 30 for capacitance detection, enabling smart diaper wetness detection based on the capacitance value, as specifically shown in FIG. 9A.

The connection terminal 31 of the detection device 30 can be a probe with a metal tip, hence it can also be called a metal tip 31. During use, it typically pierces the sensing film. To make the electrical connection between the metal tip 31 and the first metal metallization layer 11 more reliable, a locally thickened conductive layer can be provided at the corresponding position on the metal metallization layer 11, e.g., by printing conductive ink, to increase the thickness of the conductive material at that location, thereby increasing the contact area with the metal tip 31 in the thickness direction and improving connection reliability, as shown in FIG. 9B.

In FIG. 9B, a locally thickened conductive layer 11A is included on the first metal metallization layer 11. The metal tip 31 first pierces the diaper's top sheet 21, then successively pierces the locally thickened conductive layer 11A, the first metal metallization layer 11, the plastic film substrate 13, and the second metal metallization layer 12, finally reaching a corresponding elastic support (e.g., silicone) 31A. The elastic support 31A is part of the capacitance detection device 30 and provides elasticity for the contact and electrical connection between the metal tip 31 and the first metal metallization layer 11.

Regarding the situation in FIG. 9B, one might think that piercing the sensing film with the metal tip 31 would cause a short circuit between the first and second metal metallization layers 11 and 12, but this is not the case. Because the metal metallization layers of this invention are very thin (usually less than 100 nm), and because the sensing film undergoes a conical deformation at the piercing point when pierced by the metal tip, the metal tip 31 and the second metal metallization layer 12 do not make effective contact, thus avoiding a short circuit.

Even the electrical connection between the metal tip 31 and the first metal metallization layer 11 might suffer from poor contact (high or unstable contact resistance) due to the extreme thinness of the metallization layer 11. The locally thickened conductive layer 11A can significantly increase the area of contact between the detection electrode and the metal tip 31 in the thickness direction, making the electrical connection between them more stable and reliable.

If less piercing is desired, a rigid contact surface (e.g., a PCB attached to the silicone) can be added on top of the elastic support 31A, preventing the metal tip 31 from piercing into the elastic support 31A. If piercing the sensing film is undesirable, the sharpness of the metal tip 31 can be reduced (add a chamfer), allowing it to only pierce the top sheet 21 and contact the first metal metallization layer 11 without piercing through the plastic film substrate 13 of the sensing film.

Besides including a gap 26 between the second metal metallization layer 12 and the backsheet 25, another gap can be included between the first metal metallization layer 11 and the top sheet 21 if needed, allowing the first connection terminal 31 to also make face-to-face contact and electrical connection with the first metal metallization layer 11 to improve connection reliability. Conversely, if gaps are undesirable, or if electrical connection with the metal metallization layers via gap insertion is not preferred, holes/openings can be made in the backsheet or top sheet at the edge of the diaper to expose the metal metallization layer there, allowing face-to-face contact and electrical connection between the metal metallization layer and the detection device's connection terminal at the opening.

The situation where a smart diaper containing the embodiment is wetted by human excrement/ liquid containing electrolytes is shown in FIG. 10. Conductive liquid 15, including urine, feces, sweat, menstrual blood, and other human excretions, is present within the smart diaper 28. Since the liquid contains electrolytes like salts, it is conductive and can be considered a conductive liquid.

The sensing film 10 is typically placed between the diaper's top sheet and absorbent core. Liquid enters from the top sheet, flows around the sides of the sensing film, and enters the absorbent core where it is absorbed. Therefore, the width of the sensing film 10 should not be too wide, otherwise it might affect liquid penetration into the absorbent core. Since the two detection electrodes of this invention are arranged overlapping vertically, compared to prior art where two electrodes are arranged side-by-side horizontally with a gap/blank area between them, the width can be reduced by more than half, significantly reducing the impact on liquid penetration.

Another advantage of the vertical overlapping arrangement is preventing capacitive interference caused by one-sided wetness or one-sided human contact. In FIG. 10, assuming the liquid 15 is caused by sweating, since the amount of sweat is usually limited, it typically doesn't soak/wet both sides of the sensing film 10 simultaneously. Therefore, normal sweating does not generate the second capacitance, avoiding capacitive interference from sweat. Similarly, the top sheet is in direct contact with human skin. If the two detection electrodes were side-by-side on the same surface of the membrane, significant capacitive interference would occur from one-sided human contact (e.g., buttocks pressing on the diaper's top sheet). This problem does not occur in this invention; the sensing film has strong anti-interference capabilities.

When the liquid volume is large (e.g., normal urination), both sides of the sensing film 10 will be wetted simultaneously, meaning liquid will be present on both the first and second metal metallization layers 11, 12, and the liquid on these layers will connect, forming an equipotential body. In this case, capacitance C can be detected between the first and second metal metallization layers 11, 12. Capacitance C includes the first capacitance C1 generated by the membrane's structure and the second capacitance C2 generated by liquid wetting.

In the wet/wetted state, the second capacitance C2 is much larger than the first capacitance C1, so C1 can be neglected, and C can be directly considered as C2. Thus, detecting capacitance C indicates the wetness degree of the diaper.

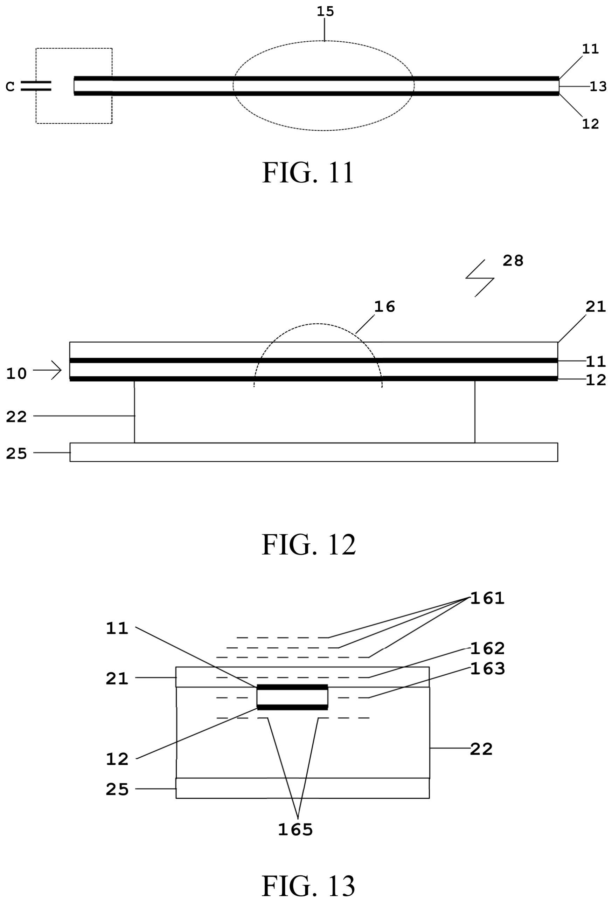

Excrements with different fluidities wet the diaper differently. A schematic B-B' longitudinal section and equivalent circuit of the diaper containing high-fluidity excrement (e.g., urine 15) are shown in FIG. 11. Due to the high fluidity of urine, it usually wets both the first metal metallization layer 11 and the second metal metallization layer 12 of the sensing film 10 simultaneously, and the degree of wetting on both sides is similar.

Schematic B-B' longitudinal and C-C' cross-sectional views of the diaper containing low-fluidity excrement (e.g., loose stool 16) are shown in FIGS. 12 and 13, respectively. Due to the poor fluidity of loose stool 16, a significant portion accumulates on the top sheet 21 (as shown at 161), then some liquid penetrates into the top sheet (162), while some flows around the sensing film 10 into the absorbent core 22 (163). The liquid 165 that reaches below the second metal metallization layer 12 of the sensing film is usually less than the liquid 162 above the first metal metallization layer 11. This situation reduces the ability of the sensing film 10 to generate the second capacitance C2, or affects its ability to detect wetness on the diaper's top sheet 21.

To solve this problem, a covering layer that can generate greater capacitance (increased sensitivity) can be added on the second metal metallization layer 12. This ensures that even if the liquid 165 reaching below the second metal metallization layer 12 is less than the liquid 162 above the first metal metallization layer 11, the capacitance generated between the second metal metallization layer 12 and the liquid 165 is not less than that generated between the first metal metallization layer 11 and the liquid 162. This prevents affecting wetness detection on the top sheet 21, or enables directional wetness detection focused towards the diaper's top sheet 21.

Another solution is to add a covering layer on the first metal metallization layer 11 that reduces capacitance generation (decreased sensitivity). This also ensures, when liquid below layer 12 is less than above layer 11, that the capacitance generated by layer 12 is not less than that generated by layer 11. Similarly, this prevents affecting wetness detection on the top sheet 21, enabling directional detection focused towards the top sheet.

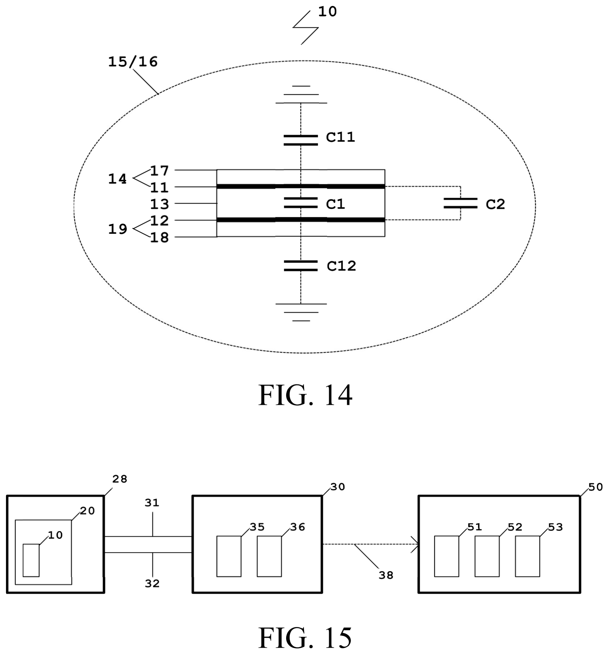

This means the sensing film can achieve directional detection or targeted wetness detection by altering the detection sensitivity on its upper and lower surfaces. A cross-sectional structural schematic of the capacitive double-sided sensing film including covering layers that alter detection sensitivity on the first and second metal metallization layers 11, 12 is shown in FIG. 14.

The difference from FIG. 3 is that an insulating covering layer (e.g., polymer material coating, silicon dioxide metallization layer, etc.) or a semiconductor covering layer (e.g., metal oxide metallization layer including aluminum oxide) 17 is included on the first metal metallization layer 11. Here, the first metal metallization layer 11 and the covering layer 17 together constitute the first detection electrode 14 of this embodiment.

Due to the presence of the covering layer 17, charged ions in the liquid/excrement 15/16 cannot directly contact the first metal metallization layer 11, reducing the generation of electric double-layer capacitance between the first metal metallization layer 11 and the liquid 15, i.e., reducing the detection sensitivity of the first detection electrode 14. In this case, the capacitance C11 between the first metal metallization layer 11 and the liquid 15 is primarily an electrolytic capacitance, where the insulating or semiconductor covering layer 17 acts as the dielectric, and the liquid in the excrement acts as the electrolyte. Its capacitance is proportional to the dielectric constant of the dielectric and inversely proportional to its thickness.

This embodiment also includes a conductive covering layer 18 on the second metal metallization layer 12, having a multi-microporous structure or made of material with high specific surface area (e.g., carbon black, graphene). Here, the second metal metallization layer 12 and the conductive covering layer 18 together constitute the second detection electrode 19 of this embodiment. The presence of the conductive covering layer 18 greatly increases the contact surface area between the second detection electrode 19 and the charged ions in the liquid 15, significantly enhancing the ability to generate electric double-layer capacitance C12 between the second detection electrode 19 and the liquid 15, i.e., greatly improving the detection sensitivity of the second detection electrode 19. The series connection of the electrolytic capacitance C11 and the electric double-layer capacitance C12 constitutes the second capacitance C2 of this embodiment.

In practical applications, an insulating covering layer, semiconductor covering layer, or conductive covering layer can be provided on any one of the first and second metal metallization layers. The insulating and semiconductor covering layers reduce the detection sensitivity of the covered side, achieving directional excrement detection focused towards the covering layer direction. The conductive covering layer increases the detection sensitivity of the covered side, achieving directional excrement detection focused towards the non-covering layer direction.

A system block diagram of the smart diaper status monitoring device formed by the sensing film 10, disposable absorbent article 20, detection device 30, and wireless receiving device 50 is shown in FIG. 15. The smart diaper 28 includes the disposable absorbent article 20, which contains the sensing film 10.

The detection device 30 includes a capacitance detection devise 35 and a wireless transmitter 36. The detection device 30 is electrically connected to the first detection electrode/first metal metallization layer and the second detection electrode/second metal metallization layer of the sensing film 10 via the first and second connection terminals 31, 32. The capacitance detection device 35 obtains capacitance information between the first and second metal metallization layers through this connection and uses this information and its variation pattern to determine the wetness status/excrement status of the smart diaper/disposable absorbent article. It then transmits the relevant status information wirelessly via the wireless transmitter 36.

The wireless status information 38 is received by the wireless receiving device 50, which includes a wireless receiving unit 51, a status display unit 52, and a status alarm unit 53. Upon receiving the status information, the wireless receiving unit 51 can display or indicate the status via the status display unit 52 or issue alarm prompts via the status alarm unit 53. Besides dedicated wireless receiving and display devices (e.g., audio-visual alarm devices), the wireless receiving device 50 can also include smartphones, personal computers, etc., running Apps or software to implement the capacitance-based wetness/excrement status detection, data recording, and query functions of this invention through a combination of hardware and software.

Since the sensing film can be cut as needed, it not only increases versatility but also significantly improves production/manufacturing efficiency. For example, wide-width sensing film webs can be produced first and then slit according to different diaper production needs, obtaining webs with specific widths suitable for particular diapers. This is a highly cost-effective production method for the sensing film.

Most materials for diaper production come in web form. The backsheet, top sheet, absorbent core, etc., are first made into webs, with each roll capable of producing thousands of diapers, reducing the frequency of loading and changing materials. For the sensing film to become a universal diaper production material, it is best also made into web form. The roll diameter of the sensing film web should not be too large, as some traditional diaper production lines might not accommodate it. A diameter not exceeding 45 cm and a length of at least 3000 meters is suitable, meaning the thickness of the sensing film must be controlled, typically within 30 micrometers.

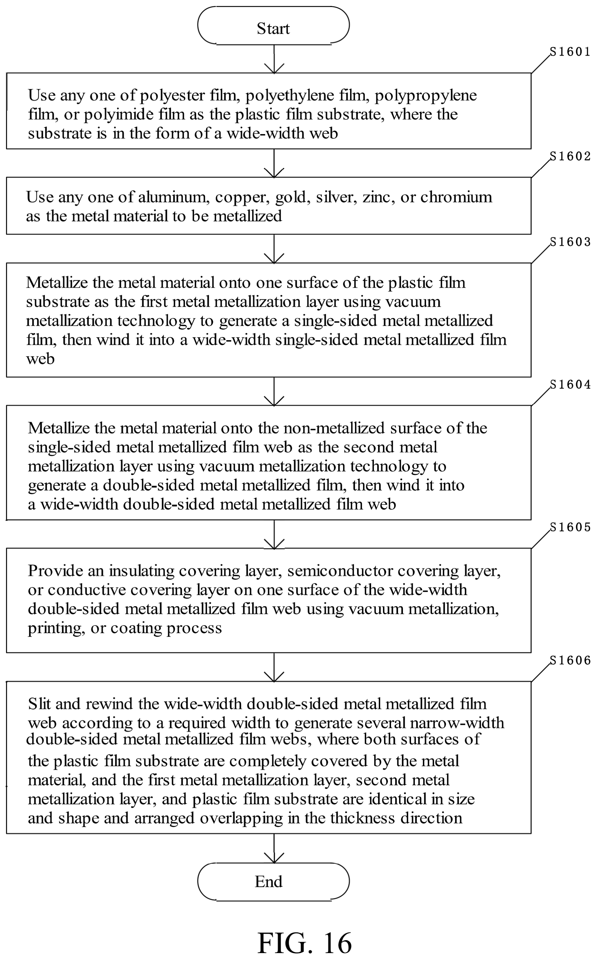

A flowchart of the method for manufacturing the sensing film is shown in FIG. 16, mainly comprising the following steps:
Step S1601: Use any one of polyester film, polyethylene film, polypropylene film, or polyimide film as the plastic film substrate, where the substrate is in the form of a wide-width web.
Step S1602: Use any one of aluminum, copper, gold, silver, zinc, or chromium as the metal material to be metallized.
Step S1603: Metallize the metal material onto one surface of the plastic film substrate as the first metal metallization layer using vacuum metallization technology to generate a single-sided metal metallized film, then wind it into a wide-width single-sided metal metallized film web.
Step S1604: Metallize the metal material onto the non-metallized surface of the single-sided metal metallized film web as the second metal metallization layer using vacuum metallization technology to generate a double-sided metal metallized film, then wind it into a wide-width double-sided metal metallized film web.
Step S1605: Provide an insulating covering layer, semiconductor covering layer, or conductive covering layer on one surface of the wide-width double-sided metal metallized film web using vacuum metallization, printing, or coating process.
Step S1606: Slit and rewind the wide-width double-sided metal metallized film web according to a required width to generate several narrow-width double-sided metal metallized film webs, where both surfaces of the plastic film substrate are completely covered by the metal material, and the first metal metallization layer, second metal metallization layer, and plastic film substrate are identical in size and shape and arranged overlapping in the thickness direction.

In the above steps, step S1605 can be omitted, proceeding directly from step S1604 to step S1606 to obtain narrow-width double-sided metal metallized film webs without insulating/semiconductor/conductive covering layers.

In the above steps, the insulating covering layer comprises a polymer material coating or a silicon dioxide metallization layer; the semiconductor covering layer comprises an aluminum oxide vacuum metallization layer; the conductive covering layer comprises a carbon-based conductive ink coating/printed layer or a graphene metallization layer.

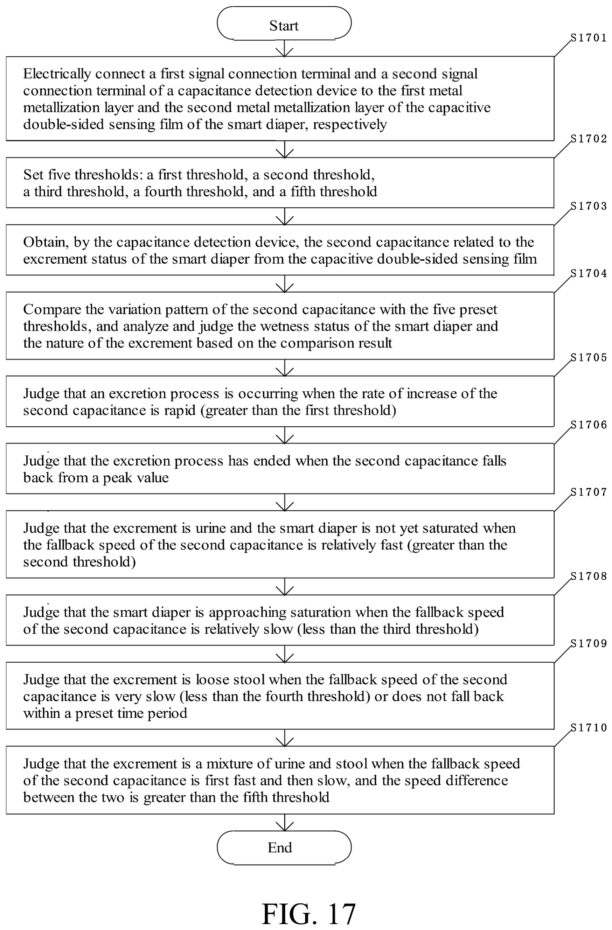

A flowchart of the method for implementing saturation detection and urine/feces differentiation on the smart diaper is shown in FIG. 17, comprising the following steps:
Step S1701: Electrically connect a first signal connection terminal and a second signal connection terminal of a capacitance detection device to the first metal metallization layer and the second metal metallization layer of the capacitive double-sided sensing film of the smart diaper, respectively.
Step S1702: Set five thresholds: a first threshold, a second threshold, a third threshold, a fourth threshold, and a fifth threshold.
Step S1703: Obtain, by the capacitance detection device, the second capacitance related to the excrement status of the smart diaper from the capacitive double-sided sensing film.
Step S1704: Compare the variation pattern of the second capacitance with the five preset thresholds, and analyze and judge the wetness status of the smart diaper and the nature of the excrement based on the comparison result.
Step S1705: Judge that an excretion process is occurring when the rate of increase of the second capacitance is rapid (greater than the first threshold).
Step S1706: Judge that the excretion process has ended when the second capacitance falls back from a peak value.
Step S1707: Judge that the excrement is urine and the smart diaper is not yet saturated when the fallback speed of the second capacitance is relatively fast (greater than the second threshold).
Step S1708: Judge that the smart diaper isapproaching saturation when the fallback speed of the second capacitance is relatively slow (less than the third threshold).
Step S1709: Judge that the excrement is loose stool when the fallback speed of the second capacitance is very slow (less than the fourth threshold) or does not fall back within a preset time period.
Step S1710: Judge that the excrement is a mixture of urine and stool when the fallback speed of the second capacitance is first fast and then slow, and the speed difference between the two is greater than the fifth threshold.

The above disclosures are only the preferred embodiments of the present invention and certainly cannot limit the scope of the rights of the present invention. Therefore, equivalent changes made according to the claims of the present invention still fall within the scope covered by the present invention.

## Claims

1. A capacitive double-sided sensing film for excrement detection, comprising:
a plastic film substrate;
a first detection electrode and a second detection electrode disposed on an upper surface and a lower surface of said plastic film substrate, respectively;
wherein said first detection electrode and said second detection electrode comprise a first metal electrode and a second metal electrode, respectively, configured to reduce internal resistance of the electrodes and a capacitance value between the electrodes and a liquid, thereby reducing the time constant of a detection circuit;
wherein said first metal electrode and said second metal electrode comprise a first metal metallization layer and a second metal metallization layer, respectively, configured to reduce thickness of the electrodes via a metal metallization process to provide required flexibility;
wherein said metal metallization layers are complete and integral vacuum metallization layers that completely cover the upper and lower surfaces of said plastic film substrate, configured to be compatible with conventional vacuum metallization processes and save cost by eliminating a need to generate circuits with specific patterns;
wherein said first metal metallization layer and said second metal metallization layer are located on the upper and lower surfaces of said plastic film substrate, respectively, and are arranged overlapping each other, configured to reduce a width of the capacitive double-sided sensing film and prevent capacitive interference caused by single-sided wetness or human body single-sided contact;
wherein said first metal metallization layer, said second metal metallization layer, and said plastic film substrate are identical in size and mutually overlapping, configured to facilitate cutting for use,
such that any cutting does not alter the identical size and mutually overlapping structural relationship;
wherein said first detection electrode, said second detection electrode, and said plastic film substrate together constitute a parallel plate capacitor and generate a first capacitance, configured to provide information related to an area of the capacitive double-sided sensing film, determine a range and a scale of signal output, thereby facilitating adaptive operation and implementing damage compensation; and
wherein when excrement containing electrolytes wets and connects at least a portion of the upper and lower surfaces of the sensing film together, a second capacitance positively correlated with a wetted area is generated between said first detection electrode and said second detection electrode,
configured to provide status information about the capacitive double-sided sensing film being wetted by the excrement.

2. The capacitive double-sided sensing film according to claim 1, **characterized in that**:
a magnitude of said first capacitance is proportional to an area and a dielectric constant of said plastic film substrate and inversely proportional to a thickness of said plastic film substrate, and is independent of a shape of the capacitive double-sided sensing film and a wetting state thereof;
said second capacitance is a series value of a capacitance generated between said first detection electrode and the excrement and a capacitance generated between said second detection electrode and the excrement, configured to ascertain an excrement wetting state of the capacitive double-sided sensing film and a nature of the excrement based on analysis of a magnitude and a variation pattern of the series value; and
said metal metallization layers contain no covering layers or holes that affect conductivity thereof, allowing electrical connection with a capacitance detection device at any position, thereby improving versatility and ease of use of the capacitive double-sided sensing film.

3. The capacitive double-sided sensing film according to claim 1, **characterized by** further comprising:
a semiconductor covering layer or an insulating covering layer covering an outer surface of any one of said metal metallization layers, configured to reduce a capacitance value between the respective metal metallization layer and the excrement, thereby achieving directional excrement detection functionality focused towards a covering layer direction; or
a conductive covering layer having a multi-microporous structure or a rough surface covering an outer surface of any one of said metal metallization layers, configured to increase a capacitance value between the respective metal metallization layer and the excrement, thereby achieving directional excrement detection functionality focused towards a non-covering layer direction; or
a locally thickened conductive layer covering a specific position on an outer surface of any one of said metal metallization layers, configured to increase a thickness of conductive material at that location, so that when electrical connection is made at that location with the metal metallization layer by a metal tip piercing method, additional conductivity is provided in a thickness direction to improve reliability of the electrical connection.

4. The capacitive double-sided sensing film according to claim 3, **characterized in that**:
said semiconductor covering layer comprises a metal oxide metallization layer, said insulating covering layer comprises an insulating material coating or metallization layer, both configured to prevent charged ions in the excrement from directly contacting the outer surface of said metal metallization layer, thereby reducing generation of said second capacitance;
said conductive covering layer comprises a carbon-based conductive ink covering layer or a graphene metallization layer, configured to increase a surface area in contact with the excrement, thereby increasing generation of said second capacitance; and
said locally thickened conductive layer comprises a conductive ink coating or printed layer.

5. The capacitive double-sided sensing film according to any one of claims 1 to 4, **characterized in that**:
said metal metallization layer comprises a vacuum aluminum metallization layer;
said plastic film substrate comprises a polyester film, polyethylene film, polypropylene film, or polyimide film; and
said excrement comprises urine, feces, sweat, or blood.

6. A smart diaper, **characterized by** comprising:
the capacitive double-sided sensing film according to claim 1; and
a disposable absorbent article comprising a top sheet, an absorbent core, and a backsheet sequentially stacked and bonded;
wherein said capacitive double-sided sensing film is disposed above said top sheet or between said top sheet and said backsheet, and is configured to provide information related to a wetting state of said disposable absorbent article through said second capacitance.

7. The smart diaper according to claim 6, **characterized in that**:
an area or a length of said capacitive double-sided sensing film has a corresponding relationship with a model specification or a length of said disposable absorbent article, configured to ascertain model specification information or length information of said disposable absorbent article by detecting said first capacitance, thereby facilitating adaptive operation.

8. The smart diaper according to claim 6, **characterized in that**:
said capacitive double-sided sensing film is disposed between said top sheet and said backsheet,
and an adhesive-free area is included between said capacitive double-sided sensing film and said top sheet or said backsheet, to facilitate electrical connection of one or both sides of said capacitive double-sided sensing film with connection terminals of a capacitance detection device, and to enable excrement detection functionality of said disposable absorbent article through the capacitance detection device; or
an opening is included on said top sheet or said backsheet, allowing one or both sides of said capacitive double-sided sensing film to be exposed through said opening and electrically connected to connection terminals of a capacitance detection device, and to enable excrement detection functionality of said disposable absorbent article through the capacitance detection device.

9. The smart diaper according to claim 8, **characterized in that**:
said capacitive double-sided sensing film is disposed between said top sheet and said absorbent core;
said absorbent core comprises a polymer absorbent material configured to gradually absorb and lock moisture entering from said top sheet, causing said top sheet to gradually become dry, such that said top sheet experiences a change process from wet to dry during each urination process, while simultaneously causing said second capacitance to experience a change process from large to small;
a speed at which said second capacitance changes from large to small is related to a saturation of said absorbent core; a higher the saturation results in a slower speed at which said second capacitance changes from large to small, wherein the saturation of said absorbent core is ascertainable based on the speed; and
when the excrement is loose stool, since loose stool is difficult for said absorbent core to absorb, said second capacitance value remains high for a long time, and this characteristic is used to determine that the excrement is loose stool.

10. A smart diaper status monitoring device, **characterized by** comprising:
the smart diaper according to claim 6; and
a capacitance detection device comprising at least two connection terminals electrically connected to said first detection electrode and said second detection electrode of said capacitive double-sided sensing film, respectively, and configured to implement excrement status monitoring functionality.

11. The smart diaper status monitoring device according to claim 10, **characterized by** further comprising:
a wireless transmitter and a wireless receiver, configured to transmit and receive excrement status information of said smart diaper, thereby implementing wireless status monitoring and status display functionality for the smart diaper.

12. A method for manufacturing the capacitive double-sided sensing film according to claim 1, **characterized by** comprising the steps of:
using any one of polyester film, polyethylene film, polypropylene film, or polyimide film as the plastic film substrate, wherein the plastic film substrate is in a form of a wide-width web;
using any one of aluminum, copper, gold, silver, zinc, or chromium as a metal material to be metallized;
metallizing the metal material onto one surface of the plastic film substrate as the first metal metallization layer by vacuum metallization technology to generate a single-sided metal metallized film, and then winding into a wide-width single-sided metal metallized film web;
metallizing the metal material onto a non-metallized surface of the single-sided metal metallized film web as the second metal metallization layer by vacuum metallization technology to generate a double-sided metal metallized film, and then winding into a wide-width double-sided metal metallized film web; and
slitting and rewinding the wide-width double-sided metal metallized film web according to a required width to generate a plurality of narrow-width double-sided metal metallized film webs, wherein upper and lower surfaces of the plastic film substrate are completely covered by the metal material, and the first metal metallization layer, the second metal metallization layer, and the plastic film substrate are identical in size and shape and are arranged overlapping in a thickness direction.

13. The method for manufacturing the capacitive double-sided sensing film according to claim 12, **characterized in that**, prior to said step of slitting and rewinding, the method further comprises a step of:
providing an insulating covering layer, a semiconductor covering layer, or a conductive covering layer on one surface of the wide-width double-sided metal metallized film web by vacuum metallization, printing, or coating process.

14. The method according to claim 13, **characterized in that**:
said insulating covering layer comprises a polymer material coating or a silicon dioxide metallization layer;
said semiconductor covering layer comprises an aluminum oxide vacuum metallization layer; and
said conductive covering layer comprises a carbon-based conductive ink coating, printed layer, or a graphene metallization layer.

15. A method for implementing saturation detection and urine/feces differentiationin the smart diaper according to claim 9, **characterized by** comprising the steps of:
electrically connecting a first signal connection terminal and a second signal connection terminal of a capacitance detection device to the first detection electrode and the second detection electrode of the capacitive double-sided sensing film of the smart diaper, respectively;
setting five thresholds comprising a first threshold, a second threshold, a third threshold, a fourth threshold, and a fifth threshold;
obtaining, by the capacitance detection device, the second capacitance related to an excrement status of the smart diaper from the capacitive double-sided sensing film;
comparing a variation pattern of the second capacitance with the thresholds, and judging a wetness status of the smart diaper and a nature of excrement based on a comparison result;
judging that an excretion process is occurring when a rate of increase of the second capacitance is greater than the first threshold;
judging that the excretion process has ended when the second capacitance falls back from a peak value;
judging that the excrement is urine and the smart diaper is not yet saturated when a fallback speed of the second capacitance is greater than the second threshold;
judging that the smart diaper is approaching saturation when the fallback speed of the second capacitance is less than the third threshold;
judging that the excrement is loose stool when the fallback speed of the second capacitance is less than the fourth threshold or when the second capacitance does not fall back within a preset time period; and
judging that the excrement is a mixture of urine and stool when the fallback speed of the second capacitance is first fast and then slow, and a speed difference between the fast and slow fallback is greater than the fifth threshold.
